# EUROPEAN PATENT SPECIFICATION

(11) **EP 2 265 316 B1**
(45) Date of publication and mention of the grant of the patent: **13.01.2016**
(21) Application number: 09716945.2
(22) Date of filing: 05.03.2009
(51) Int. Cl.: A61M 25/10

(54) **TRIGGERED DRUG RELEASE**
AUSGELÖSTE ARZNEIMITTELFREISETZUNG
LIBÉRATION DE MÉDICAMENT DÉCLENCHÉE

(30) Priority: 06.03.2008 US 34217 P
(43) Date of publication of application: 29.12.2010
(73) Proprietor: Boston Scientific Scimed, Inc., Maple Grove, MN 55311-1566 (US)
(72) Inventor: KANGAS, Steve, Woodbury Minnesota 55125 (US); ATANASOSKA, Liliana, Edina Minnesota 55436 (US)
(74) Representative: Peterreins Schley
(86) International application number: PCT/US2009/036121
(87) International publication number: WO 2009/111608

(56) References cited:
- DE-A1-102007 036 685
- US-A- 5 304 121
- US-A1- 2003 114 791

## Description

### TECHNICAL FIELD

The present invention relates to various mechanisms and devices for triggering the release of drug from a balloon catheter.

### BACKGROUND

The systemic administration of drug agents, such as by transoral or intravenous means, treats the body as a whole even though the disease to be treated may be localized. In such a case, systemic administration may not be desirable because the drug agents may have unwanted effects on parts of the body which are not to be treated, or because treatment of the diseased part of the body requires a high concentration of drug agent that may not be achievable by systemic administration.

It is therefore often desirable to administer drug agents at localized sites within the body. Common examples include cases of localized disease (e.g., heart disease) or occluded body lumens. Various methods have been proposed for such localized drug administration. For example, U.S. Pat. No. 5,304,121 describes a method of delivering water-soluble drugs to tissue at desired locations of a body lumen wall. The method generally includes the steps of impregnating a hydrogel polymer on a balloon catheter with an aqueous drug solution, inserting the catheter into a blood vessel to a desired location, and expanding the catheter balloon against the surrounding tissue to allow the release of the drug. One of the potential drawbacks to conventional drug delivery techniques using drug-impregnated polymer coatings on balloon catheters is the possible premature diffusion of the drug out of the coating during delivery into the body. Two solutions to this problem have been proposed: the use of a removable sheath over the polymer coating, and the use of a dissolvable or meltable temporary coating over the polymer coating to protect and retain the drug agent in the coating prior to a time of desired administration at a target location. The sheath approach, however, adds considerable profile to the balloon catheter device, making access to small body lumens difficult or impracticable. Furthermore, the use of a temporary protective coating over a drug-impregnated polymer coating may place undesirable time constraints on the drug delivery procedure. Moreover, it is difficult to identify or develop temporary coatings that permit the release of the drug in a consistent and predictable manner.

In view of the potential drawbacks to conventional drug delivery techniques, there exists a need for a device and method for the controlled, localized delivery of drug agents to target locations within a mammalian body while avoiding the premature release of drug agent during delivery.

### SUMMARY

In an embodiment, the present invention provides a balloon catheter comprising a catheter body having a balloon mounted thereon, the balloon having an outer expandable portion at least partially coated with a pH sensitive coating comprising a drug and a carrier material that is water-soluble at certain pHs but water-insoluble at other pHs.

In an embodiment, the present invention provides a method of releasing drug from the surface of a balloon catheter comprising providing a balloon catheter having an outer expandable portion coated with a pH sensitive coating. The pH sensitive coating comprises a drug and a carrier material that is water-soluble at certain pHs but water-insoluble at other pHs. The method further comprises exposing the pH sensitive coating to an appropriate pH to render the carrier material water-soluble to thereby allow drug release.

In another embodiment, the present invention provides a method of preventing undesired drug release during delivery of a drug coated balloon catheter comprising providing a balloon catheter having an outer expandable portion coated with a pH sensitive coating The coating comprises a drug and a carrier material that is water-soluble at certain pHs but water-insoluble at other pHs. The method further comprises inserting the balloon catheter into a lumen and advancing the balloon catheter to the target site while the coating is water-insoluble. The method further includes exposing the pH sensitive coating to an appropriate pH once at the target site to render the carrier material water-soluble to thereby allow drug release.

In an embodiment, the present invention provides a balloon catheter comprising a catheter body having a balloon mounted thereon, the balloon having an outer expandable portion at least partially coated with a coating, the coating comprising a pH sensitive hydrogel and a drug, wherein the pH sensitive hydrogel swells at certain pHs but shrinks at other pHs.

In another embodiment, the present invention provides a method of releasing a drug from the surface of a balloon catheter comprising providing a balloon catheter having an expandable portion coated with coating comprising a pH sensitive hydrogel and a drug. The pH sensitive hydrogel swells or shrinks with change in pH. The method further comprises exposing the coating to an appropriate pH to swell the hydrogel to thereby allow drug release.

In another embodiment, the present invention provides a method of preventing undesired drug release during delivery of a drug coated balloon catheter comprising providing a balloon catheter having an expandable portion coated with coating comprising a pH sensitive hydrogel and a drug. The pH sensitive hydrogel swells or shrinks with change in pH. The method further comprises inserting the balloon catheter into a lumen and advancing the balloon catheter to the target site while the hydrogel is in a shrunken state. The method further includes exposing the pH sensitive coating to an appropriate pH once at the target site to swell the hydrogel to thereby allow drug release.

In an embodiment, the present invention provides a balloon catheter comprising a catheter body having a balloon mounted thereon, the balloon having an outer expandable portion comprising a drug conjugated to a polymer by a hydrolyzable bond.

In another embodiment, the present invention provides a method of releasing a drug from the surface of a balloon catheter comprising providing a balloon catheter having an expandable portion comprising a drug conjugated to a polymer by a hydrolyzable bond. The method further comprises hydrolyzing the bond between the drug and the polymer to release the drug.

In another embodiment, the present invention provides a method of preventing undesired drug release during delivery of a drug coated balloon catheter. The method comprises providing a balloon catheter having an expandable portion comprising a drug conjugated to a polymer by a hydrolyzable bond. The method further comprises inserting the balloon catheter into a lumen and advancing the balloon catheter to the target site. The method further comprises hydrolyzing the bond between the drug and the polymer once at the target site thereby releasing the drug into the target site.

### BRIEF DESCRIPTION OF THE DRAWINGS

**FIG. 1** is cross-sectional view of an embodiment of a balloon catheter according to the present invention.
**FIG. 2** is a cross-sectional view of an embodiment of a balloon catheter according to the present invention.
**FIG. 2a** is an enlarged cross-sectional view of the balloon portion of a balloon catheter of **FIG. 2** showing pores through which a solution can pass to contact the coating on the outer surface of the balloon.
**FIG. 3** is schematic illustration of a coating on a balloon catheter where therapeutic agent is contained within the coating.
**FIG. 4** is a schematic illustration of a coating after the coating is exposed to a triggering agent to activate the coating and release drug therefrom.
**FIG. 5** is an enlarged cross-sectional view of a balloon catheter including a semi-permeable outer balloon and inner balloon.
**FIG. 6** is a further enlarged schematic illustration of a portion of **FIG. 5** which shows the manner in which a semi-permeable balloon prevents passage of a drug.
**FIG. 7** is a further enlarged schematic illustration of a portion of **FIG. 5** which shows the manner in which a semi-permeable balloon enables passage of a drug.
**FIG. 8** is a much enlarged cross-sectional view of the region C in **FIG. 6** of the membrane, illustrating the pathways through the thickness of the membrane.

### DETAILED DESCRIPTION OF THE INVENTION

The present invention provides devices and methods for delivering drug to a target site via a medical device without the drug prematurely releasing from the medical device during transit to the target site. Specifically, the present invention provides various embodiments where drug is released from a drug coated balloon catheter via a triggering condition. The triggering condition can be, for example, a change in solubility, a change in volume, or hydrolysis. A triggering agent can bring about the triggering condition.

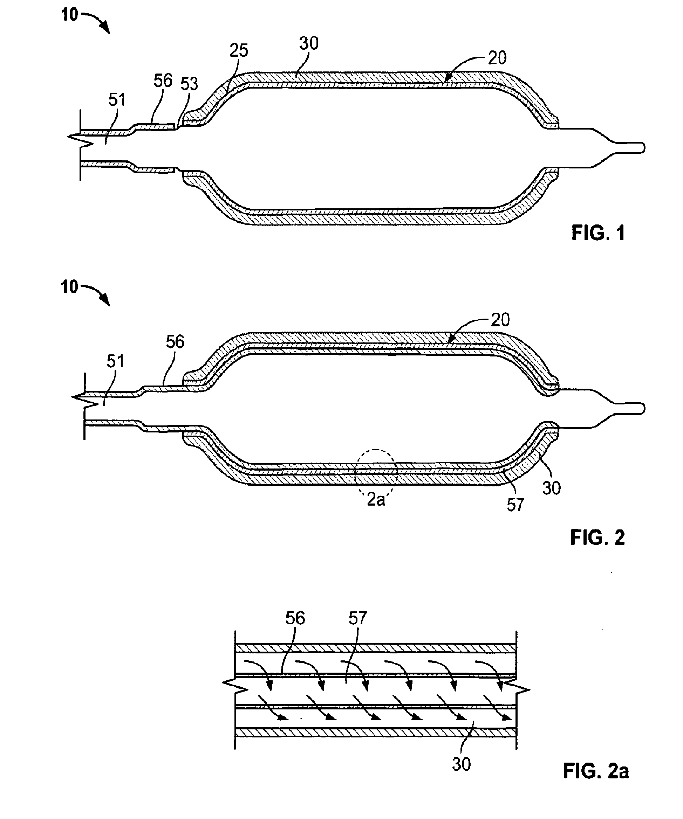

Referring to FIG. 1, in certain embodiments, a balloon catheter 10 comprises a catheter body 51 having a balloon 20 mounted thereon. Balloon 20 has an expandable portion with an outer surface 25 at least partially coated with a coating 30 comprising a carrier material comprising a therapeutic agent. A triggering agent can be delivered to coating 30 via a lumen 56 of the catheter 10. Specifically, in an embodiment, the triggering agent passes to the outer surface portion 25 of balloon 20 through opening 53 which is located proximal of the outer surface portion 25. The agent is then carried by the flow of bodily fluids towards outer surface portion 25 where the solution washes over coating 30. Contact of a triggering agent with coating 30 induces the release of therapeutic agent carried by coating 30.

Referring to FIG. 2, in another embodiment, a balloon catheter 10 comprises a balloon **20** having a porous interface **57** and a coating **30** at least partially disposed thereon which comprises a carrier material comprising a therapeutic agent. Balloon catheter **10** further comprises a lumen **56,** which is in communication with porous interface **57** of balloon **20.** Referring to **FIG. 2a****,** a triggering agent delivered through lumen **56** can exit lumen **56** through porous interface **57** to coating **30.** In the absence of lumen **56,** a triggering agent can pass directly to coating **30** through catheter **10.** Such embodiments are only exemplary and the present invention is not limited to any particular catheter configuration so long as a triggering agent can access coating **30.**

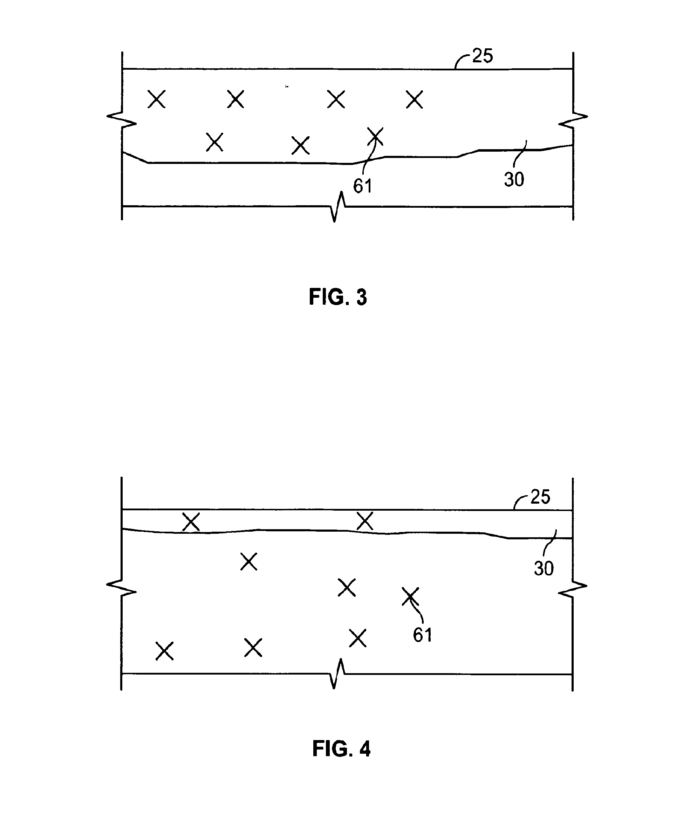

Referring to **FIG. 3** and as described in general above, a therapeutic agent **61** is carried in or on the coating **30** at least partially disposed on the outside surface **25** of balloon **20** of catheter **10.** When coating **30** is exposed to an appropriate triggering agent, the coating is "activated" to release drug therefrom as shown in **FIG. 4****.**

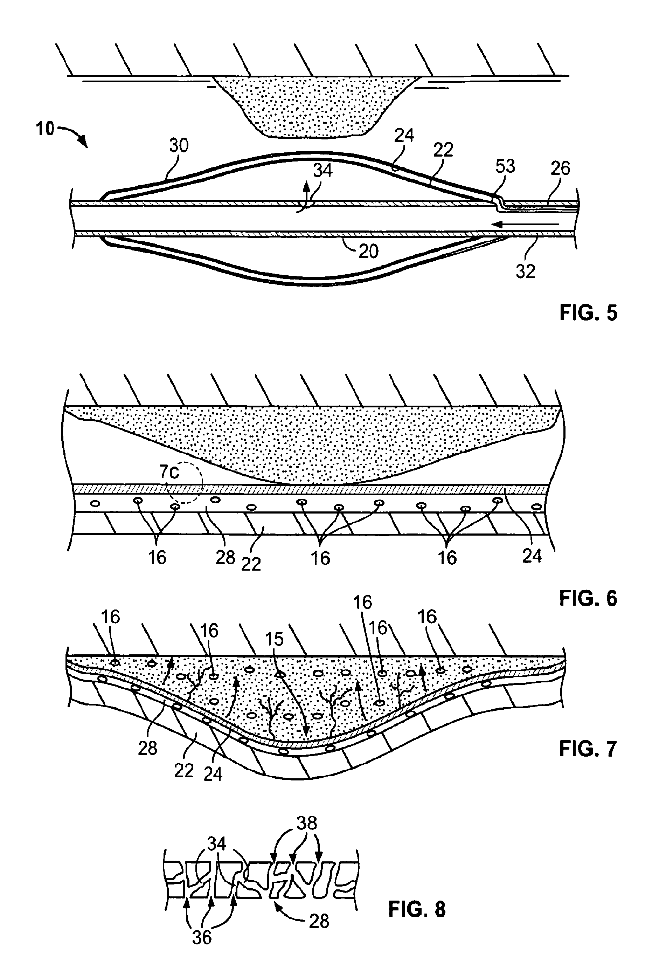

Referring to **FIGs**. **5** and **6****,** in certain embodiments, a triggering agent is delivered to a coating on the outside of a balloon catheter using a double membrane catheter. For example, a catheter **10** can carry an inner balloon **22** and a porous outer balloon **24,** through which the a triggering agent may pass to coating **30** on outer balloon **24** under conditions of pressure. Coating **30** can comprise a drug immobilized therein until released by exposure to a triggering agent. The triggering agent can be introduced through a first lumen **26** into an intermediate region **28** (illustrated in **FIG. 6****)** between balloons **22** and **24** via a first aperture **53.** At the time of dilatation, inflation fluid passes through a second lumen **32** and through port **34** to inflate inner balloon **22.** Inflation of inner balloon **22** provides the pressure needed on the solution comprising a triggering agent in intermediate region **28** to effect passage of the solution through openings of porous outer balloon **24.** After release of the drug from coating **30** is triggered by the triggering agent, inner balloon **22** can be deflated and intermediate region **28** can be re-filled with drug, which can then be delivered to coating **30** to replenish it. The process of replenishing coating **30** with drug and delivering a triggering agent to coating **30** to induce the release of drug from coating **30** may be repeated as many times as desired.

The inner balloon may be of the type used in dilatation of blood vessels and made, for example, of a somewhat compliant material such as polyethylene that conforms to the shape of the body lumen wall or a nondistendable material such as PET. At least a portion of the outer balloon may include a membrane selected to create sufficient resistance so that the triggering agent or drug weeps out of the membrane and into the coating. This gentle application is advantageous since injury or disruption of the vessel wall is avoided. Preferably, the membrane has at least one layer of hydrophobic material having small openings, for example, 2.20 microns, to create a large pressure drop across the membrane to dissipate the pressure applied to the triggering agent in the intermediate region during balloon inflation and effect a low-energy weeping delivery of the triggering agent. Prior to pressuring the triggering agent, the small openings can prevent the flow of the triggering agent from the intermediate region (or the contamination of the intermediate region with body fluid from the body lumen) as further discussed below.

Referring to **FIG. 8****,** porous outer balloon **24** can include a series of tortuous paths **34** through its thickness connecting openings **36** on its inner surface exposed to the region **28** and openings **38** exposed to the body lumen. The flow of triggering agent through the pathways can reduce the velocity of the triggering agent and enables gentle application to the coating and then to the tissue. Suitable materials for the porous outer balloon or a porous membrane of the outer balloon include, but are not limited to, GORETEX™ (an expanded porous fluorocarbon plastic material) and ultrahigh molecular weight microporous polyethylene (a polyethylene material available through Millipore, Inc. and commonly used for filter membranes). Either the entire balloon **24** is formed from the semi-permeable material or a patch of the material is attached to a normal balloon e.g. by heat sealing. Other semi-permeable membranes may be formed by providing relatively large, substantially straight pathways through a hydrogel material and applying thereover, on the outside of the membrane, a hydrogel, e.g., of polyacrylic acid of the type described in U.S. Ser. No. 297,331, filed Jan. 17, 1989. Low energy application may also be achieved by a porous material formed of a series of layers having offset openings or a series of woven layers, which create a tortuous pathway for passage of a solution.

Preferably, the semi-permeable material is hydrophobic with openings of a size selected to prevent substantial flow of solutions from the region **28** until sufficient pressure is applied. Referring to **FIGs. 6** and **7****,** the water intrusion pressure of the permeable hydrophobic material and that of the solution are selected such that the solution will not normally pass through the openings unless sufficient pressure is applied by inner balloon **22.** Selection of the proper intrusion pressure for the semi-permeable material of outer balloon **24** prevents passage of the drug or triggering agent **16** through the openings of outer balloon **24** when the balloon is less than fully inflated.

As demonstrated in **FIG. 7****,** under conditions of sufficiently high intrusion pressure (e.g., with the balloon fully inflated), the triggering agent or drug **16** passes through the openings in a low-pressure, low-energy, non-injurious manner. Inner balloon **22** may be deflated, and the device may be refilled with a triggering agent, if necessary, and moved to various locations, where inner balloon **22** is re-inflated to deliver the triggering agent or drug, or another drug altogether to the various locations. This process may be repeated. A constant pressure pump may be used to maintain the balloon pressure above that needed to administer a solution through the openings during treatment. It will be understood that the amount of the triggering agent or drug delivered may also be carefully controlled by application of sufficient pressure to enable administration, then reducing the pressure below the threshold for passage through the openings, at which point, delivery of solution to the coating ceases.

The procedure can be performed in many body lumens, most preferably, the vascular system in which case dilatation of a stenosed blood vessel may be carried out before, after or simultaneously with drug application.

In certain embodiments, a coating of a balloon catheter is a pH sensitive coating comprising therapeutic agent dispersed or otherwise contained within a carrier material that is water-soluble at certain pHs but water-insoluble at other pHs. Non-limiting examples of such carrier materials include fatty acids and citric acid that are water-soluble at basic pHs but water-insoluble at neutral or acidic pHs, bases such as amines that are water-soluble at acidic pHs but water-insoluble at neutral or basic pHs, and polymers of which the solubility changes with pH. Non-limiting examples of such polymers include sulfonamide-based polymers and copolymers, amine functional polymers such as polyvinyl pyridine polymers and copolymers, and polysaccharides such as chitosan that are water-soluble at acidic pHs but water-insoluble at neutral or basic pHs and poly (vinylpyrrolidone-co-dimethylmaleic anhydride) (PVD) that is water-soluble at neutral and acidic pHs but water-insoluble at basic pHs. One of ordinary skill in the art could readily determine other carrier materials that are water-insoluble at certain pHs but water-soluble at other pHs. The drug can be dispersed or otherwise placed in the carrier material at a pH at which the carrier material is water-insoluble, for example, and the resultant coating can be applied to the at least a portion of the outer expandable portion of the balloon catheter. The balloon catheter can then be inserted into a lumen of the body and delivered to the target site. During the delivery, the carrier material can be kept water-insoluble. Once at the target site, the balloon can be inflated, and the pH can be changed to render the carrier material water-soluble, thereby allowing release of the drug. For example, when an acid or a base is used as a carrier, the pH change causes formation of a salt, which renders the carrier material water-soluble,. When a polymer is used as a carrier, because the polymer dissolves at certain pHs, upon exposure to the appropriate pH and release of the drug, the polymer dissolves in the bloodstream.

In certain embodiments, a coating of a balloon catheter is a coating comprising a pH sensitive hydrogel and therapeutic agent dispersed or otherwise contained within the hydrogel. The volume of the hydrogel changes, i.e. the hydrogel swells or shrinks, with pH. Non-limiting examples of a suitable hydrogel include carboxylic acid functional polymers such as acrylic polymers incorporating acrylic and/or methacrylic acid, poly(ethylene imine), poly(propylene imine), chitosan, poly(L-lysine), and poly(L-histidine). One of ordinary skill in the art could readily determine other hydrogels that are pH sensitive. The drug can be dispersed or otherwise placed in the hydrogel before or after the hydrogel-containing coating is applied to the outer expandable portion of the balloon catheter. The balloon catheter can then be inserted into a lumen of the body and delivered to the target site. During the delivery, the hydrogel can be kept at its shrunken state. Once at the target site, the balloon can be inflated, and the pH can be changed to swell the hydrogel, thereby allowing release of the drug.

In another embodiment, the triggering condition is hydrolysis and the triggering agent is an agent capable of hydrolyzing a drug. For example, in certain embodiments, a drug is attached to an outer expandable portion of a balloon catheter via a hydrolyzable bond, such as an ester bond, such that the hydrolyzable bond is cleaved by exposure to a triggering agent. One way of achieving this is by conjugating a drug with a hydroxyl group to a polymer on the surface of the balloon with a free carboxylic acid group to form an ester bond between the carboxylic acid of the polymer and the hydroxyl group of the drug. Alternatively, the expandable portion itself is fabricated from a polymer that can form a hydrolyzable bond with a drug. Non-limiting examples of such a polymer include polyglutamic acid and polylactic acid. The balloon catheter can then be inserted into a lumen of the body and delivered to the target site. During the delivery, the hydrolyzable bond can be kept intact. Once at the target site, the balloon can be inflated, and the bond is hydrolyzed, thereby allowing release of the drug. The bond could be hydrolyzed in a number of ways, such as, for example, exposure to a catalyst or a change in pH around the ester bond. Regarding exposure to a catalyst, a non-limiting example of a catalyst is polyoxomethylate. The catalyst can also be an enzyme found in the body such as an esterase, which is found in blood. The catalyst can be delivered to the surface of the outer expandable portion of the balloon catheter via a lumen as described above.

A change in pH for any of the embodiments described above could be initiated chemically by delivering a basic or acidic solution to the site or electrochemically by water electrolysis. For electrochemical pH change, an electrode can be the cutting blades on a balloon catheter or the entire or partial surface of the balloon coated with a conductive material. Non-limiting examples of such a conductive material include metals, such as gold and platinum, and conductive polymers such as polypyrrole, poly (3,4-ethylene-dioxythiphene) (PEDOT), poly acetylene, and polyaniline. A current could be applied anodically at the site to electrochemically convert water surrounding the balloon to oxygen gas and hydrogen ions, thereby creating an acidic condition. Alternatively, a current could be applied cathodically at the site to electrochemically convert water surrounding the balloon to hydrogen gas and hydroxyl ions, thereby creating a basic condition. The current could be applied during the delivery of the balloon catheter to keep the local pH at a desired level to prevent the release of the drug. The current could be applied at the target site to obtain a desired pH to release the drug.

The therapeutic agent that is applied to the outer expandable portion of a balloon catheter of the present invention can be any suitable biologically acceptable agent such as a non-genetic therapeutic agent, a biomolecule, a small molecule, or cells. For example, the therapeutic agent can be pharmaceutically active compound. Non-limiting examples of therapeutic agents include anti-thrombogenic agents, anti-proliferative agents, anti-inflammatory agents, antineoplastic/anti-proliferative/anti-mitotic agents, anti-microbial agents, biofilm synthesis inhibitors, antibiotics, antibodies, anesthetic agents, nitric oxide, nitric oxide (NO) donors, anticoagulants, vascular cell growth promoters, vascular cell growth inhibitors, cholesterol-lowering agents, vasodilating agents, agents which interfere with endogenous vasoactive mechanisms, inhibitors of heat shock proteins angiotensin converting enzyme (ACE) inhibitors, beta-blockers, βAR kinase (βARK) inhibitors, phospholamban inhibitors, protein-bound particle drugs, and any suitable combination thereof. Non-limiting examples of specific drugs include taxol, enoxaparin, sirolimus, tacrolimus, everolimus, zotarolimus, angiopeptin, and combinations thereof.

The foregoing description and examples have been set forth merely to illustrate the invention and are not intended as being limiting. Each of the disclosed aspects and embodiments of the present invention may be considered individually or in combination with other aspects, embodiments, and variations of the invention. Further, while certain features of embodiments of the present invention may be shown in only certain figures, such features can be incorporated into other embodiments shown in other figures while remaining within the scope of the present invention. In addition, unless otherwise specified, none of the steps of the methods of the present invention are confined to any particular order of performance.

## Claims

1. A balloon catheter comprising:
a catheter body having a balloon mounted thereon, the balloon having an outer expandable portion at least partially coated with a conductive material, functioning as at least one electrode, and at least partially coated with a pH sensitive coating containing a drug.

2. The balloon catheter of claim 1, wherein the pH sensitive coating comprises a pH sensitive hydrogel and a drug, wherein the pH sensitive hydrogel swells at certain pHs but shrinks at other pHs.

3. The balloon catheter of claim 2, wherein the pH-sensitive hydrogel is selected from acrylic polymers incorporating acrylic and/or methacrylic acid, poly(ethylene imine), poly(propylene imine), chitosan, poly(L-lysine), and poly(L-histidine).

4. The balloon catheter of claim 1, wherein the pH sensitive coating comprises a drug conjugated to a polymer by a bond hydrolyzed upon exposure to a change in pH.

5. The balloon catheter of claim 4, wherein the outer expandable portion of the balloon catheter is fabricated from the polymer.

6. The balloon catheter of claim 4, wherein the bond is an ester bond.

7. The balloon catheter of claim 4, wherein the polymer is polyglutamic acid or polylactic acid.

8. The balloon catheter of claim 1, wherein the pH sensitive coating comprising a drug and a carrier material, the carrier material being water-soluble at certain pHs but water-insoluble at other pHs.

9. The balloon catheter of claim 8, wherein the carrier material is a fatty acid or citric acid, an amine or a compound with at least one amine functional group or a polymer.

10. The balloon catheter of claim 9, wherein the carrier material is an amine or a compound with at least one amine functional group.

11. The balloon catheter of claim 9, wherein the carrier material is a polymer.

## Patentansprüche

1. Ein Ballonkatheter umfassend:
einen Katheterkörper mit einem darauf befestigten Ballon, wobei der Ballon einen äußeren expandierbaren Teil aufweist, der mit einem leitfähigen als mindestens eine Elektrode fungierenden Material beschichtet ist, und wobei er zumindest teilweise mit einer einen Arzneistoff enthaltenden pH-empfindlichen Beschichtung beschichtet ist.

2. Der Ballonkatheter nach Anspruch 1, wobei die pH-empfindliche Beschichtung ein pH-empfindliches Hydrogel und einen Arzneistoff umfasst, wobei das pH-empfindliche Hydrogel bei gewissen pHs aufquellt aber bei anderen pHs schrumpft.

3. Der Ballonkatheter nach Anspruch 2, wobei das pH-empfindliche Hydrogel gewählt ist unter Acrylpolymeren, einschließend Acryl- und/oder Methacrylsäure, Poly(ethylenimin), Poly(propylenimin), Chitosan, Poly(L-lysin) und Poly(L-histidin).

4. Der Ballonkatheter nach Anspruch 1, wobei die pH-empfindliche Beschichtung einen Arzneistoff umfasst, der an ein Polymer durch eine Bindung konjugiert ist, welche bei Exponierung gegenüber einer pH-Veränderung hydrolysiert wird.

5. Der Ballonkatheter nach Anspruch 4, wobei der äußere expandierbare Teil des Ballonkatheters aus dem Polymer hergestellt ist.

6. Der Ballonkatheter nach Anspruch 4, wobei die Bindung eine Esterbindung ist.

7. Der Ballonkatheter nach Anspruch 4, wobei das Polymer Polyglutaminsäure oder Polylactid ist.

8. Der Ballonkatheter nach Anspruch 1, wobei die pH-empfindliche Beschichtung einen Arzneistoff und ein Trägermaterial umfasst, wobei das Trägermaterial bei gewissen pHs wasserlöslich ist aber bei anderen pHs wasserunlöslich ist.

9. Der Ballonkatheter nach Anspruch 8, wobei das Trägermaterial eine Fettsäure oder Zitronensäure, ein Amin oder eine Verbindung mit mindestens einer aminfunktionellen Gruppe, oder ein Polymer ist.

10. Der Ballonkatheter nach Anspruch 9, wobei das Trägermaterial ein Amin oder eine Verbindung mit mindestens einer aminfunktionellen Gruppe ist.

11. Der Ballonkatheter nach Anspruch 9, wobei das Trägermaterial ein Polymer ist.

## Revendications

1. Cathéter à ballonnet comprenant :
un corps de cathéter ayant un ballonnet monté sur celui-ci, le ballonnet ayant une partie expansible extérieure au moins en partie revêtue d'un matériau conducteur, fonctionnant en tant qu'au moins une électrode, et au moins en partie revêtue d'un revêtement sensible au pH contenant un médicament.

2. Cathéter à ballonnet selon la revendication 1, dans lequel le revêtement sensible au pH comprend un hydrogel sensible au pH et un médicament, l'hydrogel sensible au pH gonflant à certains pH mais se contractant à d'autres pH.

3. Cathéter à ballonnet selon la revendication 2, dans lequel l'hydrogel sensible au pH est choisi parmi des polymères acryliques incorporant de l'acide acrylique et/ou méthacrylique, du poly(éthylène imine), du poly(propylène imine), du chitosane, du poly(L-lysine), et du poly(L-histidine).

4. Cathéter à ballonnet selon la revendication 1, dans lequel le revêtement sensible au pH comprend un médicament conjugué à un polymère par une liaison hydrolysée lors de l'exposition à un changement de pH.

5. Cathéter à ballonnet selon la revendication 4, dans lequel la partie expansible extérieure du cathéter à ballonnet est fabriquée à partir du polymère.

6. Cathéter à ballonnet selon la revendication 4, dans lequel la liaison est une liaison ester.

7. Cathéter à ballonnet selon la revendication 4, dans lequel le polymère est de l'acide polyglutamique ou de l'acide polylactique.

8. Cathéter à ballonnet selon la revendication 1, dans lequel le revêtement sensible au pH comprend un médicament et un matériau support, le matériau support étant hydrosoluble à certains pH mais non hydrosoluble à d'autres pH.

9. Cathéter à ballonnet selon la revendication 8, dans lequel le matériau support est un acide gras ou un acide citrique, une amine ou un composé ayant au moins un groupe fonctionnel amine ou un polymère.

10. Cathéter à ballonnet selon la revendication 9, dans lequel le matériau support est une amine ou un composé ayant au moins un groupe fonctionnel amine.

11. Cathéter à ballonnet selon la revendication 9, dans lequel le matériau support est un polymère.
